# EUROPEAN PATENT SPECIFICATION

(11) **EP 2 194 907 B1**
(45) Date of publication and mention of the grant of the patent: **10.10.2012**
(21) Application number: 08788972.1
(22) Date of filing: 01.08.2008
(51) Int. Cl.: A61B 19/02, A61B 19/08, A61B 19/10, A61F 13/00, A61F 13/02, A61F 15/00, A61M 25/02

(54) **CATHETER CLIP**
KATHETERKLAMMER
DISPOSITIF DE FIXATION D'UN CATHÉTER

(30) Priority: 27.08.2007 US 966428 P
(43) Date of publication of application: 16.06.2010
(73) Proprietor: Jaymore Enterprises Limited, Limassol (CY)
(72) Inventor: PANOTOPOULOS, Christos, 11521 Athens (GR)
(74) Representative: Bösl, Raphael Konrad
(86) International application number: PCT/IB2008/002009
(87) International publication number: WO 2009/027778

(56) References cited:
- WO-A-97/21459
- WO-A-03/068304
- US-A- 3 900 026
- US-A- 4 869 719
- US-B1- 6 682 506

## Description

### FIELD OF THE INVENTION

The invention generally relates to a device according to the preamble of claim 1, for securing an invasive medical implementation to a subject. Specific embodiments allow a user to secure a catheter to a patient rapidly and without the aid of prior surgical tapes, glues, dressings and/or foams.

### BACKGROUND

Medical implementations such as catheters, medical lines, tubing and like articles are routinely used to move fluids to and from patients. A catheter typically includes a hard part which remains exterior to the patient, and a soft part, at least a portion of which is inserted into the patient. The hard part can include a connection point (sometimes called a hub) to which other medical implementations (eg., a syringe, fluid supply tube) can be joined. Following installation and securement ("catheterization"), the catheter is a convenient means for administering fluids such as drugs or withdrawing blood or other body fluids from the patient.

According to prior practice, a caregiver uses glue, foam, surgical tape, dressing or a combination thereof to secure the catheter against the skin of the patient. Typically, the caregiver covers the catheter insertion site with a dressing after swabbing the area with antiseptic. The entire procedure can take several minutes or more. In addition, catheterization can require frequent disconnection of the catheter as new medical lines are added or replaced, thereby stressing securing tapes, glues or foams. In settings involving long-term catheter use, the caregiver must frequently clean the insertion site about the inserted (indwelling) catheter, change the dressings, and apply fresh antiseptic.

There has been increasing recognition that caregivers spend too much time securing catheters to patients. Moreover, prior practice has not been able to prevent catheter dislodgement and/or infection near insertion sites. Catheters that are taped or glued in place are readily pulled out during "routine" dressing changes. Surgical tapes and foams can be uncomfortable or irritating for some. Many patients cannot rest comfortably knowing that a secured catheter may dislodge from the insertion time during sleep. Young or elderly patients are especially vulnerable to these and related shortcomings.

WO 97/21459 A discloses a device for fixating a drainage tube relative to a skin surface part of a patient. The device comprises a plaster component having a central aperture and carrying an adhesive layer on the lower side thereof by means of which the plaster component adheres to the skin surface of the patient to whom the device is applied. After the plaster component has been fixated to the body of the patient, the drainage tube is bent to a position in which it extends substantially parallel to the skin surface of the patient and in contact with the skin surface. The tube is then taken up by a groove. A securing strip is then moved to a position in which a lower side of the securing strip is in contact with an upper surface of a support component of the device and the drainage tube. In this way, the drainage tube is secured and maintained in place.

It is becoming clear that prior catheter securement devices and procedures have not optimally served patients. These and other drawbacks have created a need for a more rapid and reliable device for securing catheters to patients. Accordingly, it is the object of the invention to provide a device that can be used to secure a catheter to the skin of a patient that does not rely on use of prior tapes, glues, dressings and/or foams.

This object is achieved by a device according to claim 1.

### SUMMARY

In broad terms, the invention provides a device for securing an invasive medical implementation such as a catheter to a patient. The device generally combines (1) an adherent surface that attaches the device to the skin and (2) a flexible clasping means to grasp the catheter and secure it to the device. Securement is typically reversible. Preferred practice of the invention avoids use of prior tapes, glues, dressings and/or foams to secure the medical implementation to the patient. Use of the invention can substantially reduce catheter securement times, thereby enhancing the reliability, comfort, and safety of catheterization. The invention is relatively simple to use and can be employed by experienced and inexperienced caregivers alike.

Accordingly, the invention provides a device having the features of claim 1, for securing a catheter to an insertion site of a patient.

The invention further provides a unitary package that includes the device wherein the device is preferably sterile and includes a packing material substantially resistant to penetration by microorganisms, viruses and the like.

The invention can be used in a method not covered by the invention, of securing a catheter to an insertion site of a patient, wherein the method includes at least one of and preferably all of the following steps:
removing, with at least one digit, the seal from the patient contacting surface of the flexible portion to expose the adhesive,
guiding the device over the catheter,
contacting the flexible base (patient contacting surface) to the patient sufficient to adhere the device thereto; and
actuating the clasping means to grasp the catheter (preferably at or around the solid part), thereby securing the catheter to insertion site of the patient.

Further uses and advantages of the invention will be apparent from the following Drawings and discussion.

### BRIEF DESCRIPTION OF THE DRAWINGS

Figure 1A is a drawing (top view) showing a catheter clip of the invention.
Figure 1B is a drawing (top view) showing the catheter clip securing a catheter having lateral wings for stabilizing the catheters and connecting hubs.
Figure 2 is a drawing (cross-sectional view) of the catheter clip securing a catheter against the skin of a patient.

### DETAILED DESCRIPTION

In the present section, the invention is illustrated with regard to one or more particular embodiments. These embodiments are intended merely to illustrate certain principles of the invention and not to limit the invention or use thereof in any way.

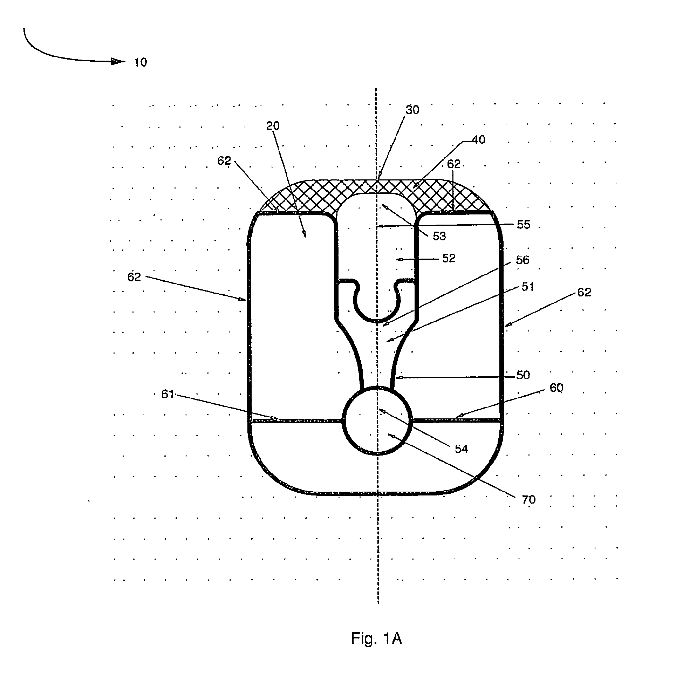

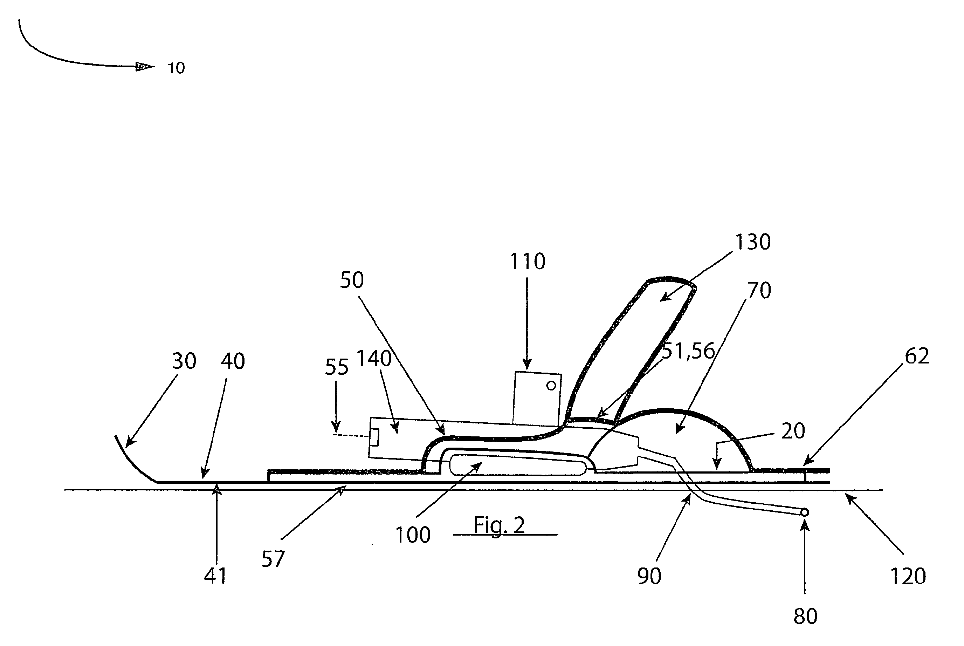

Figure 1A shows a particular embodiment of the invention ("catheter clip") for securing a catheter to a skin insertion site of a patient. The catheter clip **10** includes a solid portion **50** joined to a flexible base **20**. The flexible base **20** includes an underlying patient contacting surface **41** (see Fig. 2) that is at least partially sealed with a suitable sealing medium such as paper or a plastic. The solid portion **50** includes a clasping means **51** adapted to grasp and securing the catheter to the catheter clip **10**. The clasping means **51** may include or consist of nearly any suitable plastic material provided it flexibly resists extension by a user. A cover **40** is in actuating contact with a handle **30** attached to the flexible base **20**. The handle **30** and the cover **40** are in sealing contact with a seal formed on at least part of the patient contacting surface **41**. Preferably, the seal will protect an adhesive on at least part of the patient contacting surface **41**, preferably substantially all of it. The amount of adhesive on the patient contacting surface **41** is not critical provided it is sufficient to attach the catheter clip **10** to the patient.

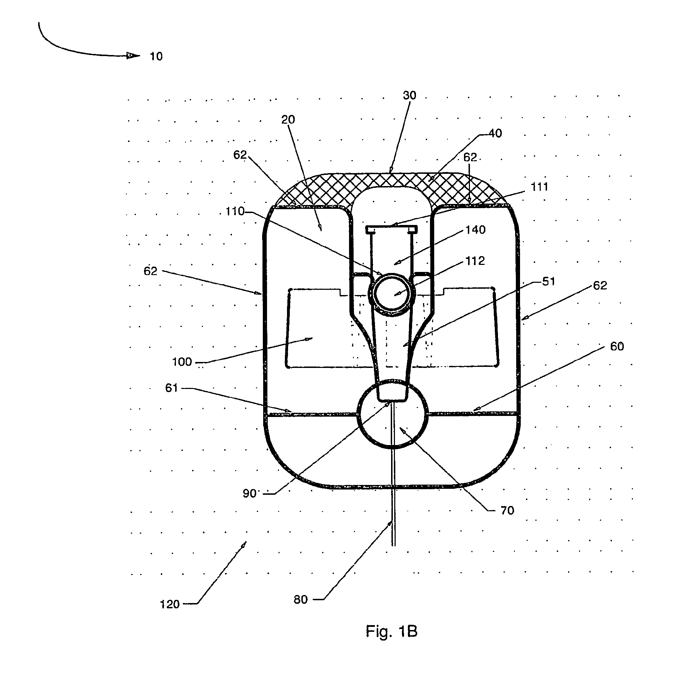

Referring again to Figure 1A, the solid portion **50**, flexible base **20** and the handle **30** together define an open chamber **52** that includes a distal end **53** (shown contacting a first centerpoint) for receiving the catheter and a proximal end **54** (shown contacting a second centerpoint) for accepting the catheter from the distal end **53** toward the skin insertion site **90** (see Fig.1B). The first centerpoint of the distal end **53** and the second centerpoint of the proximal end define an axis **55** surrounded by the open chamber **52**. In preferred use, a catheter (typically the hard part) is placed along the axis **55** for securement to the catheter clip **10** by the clasping means **51**. As shown, the open chamber **52** further includes a top **56** defined by at least part of the solid portion **50** and a bottom **57** (see Fig. 2) opposite the top **56** and defined by the handle **30** and the flexible base **20**. In the embodiment shown, the top **56** is in actuating contact with the clasping means **51**, wherein actuation of the top **56** is sufficient to flex the clasping means **51** so that it grasps and secures the hard part of a catheter to the catheter clip **10**.

The flexible base **20** and optionally the solid portion **50** further define a compartment **70** positioned at or near the insertion site **90**. The compartment **70** is typically adapted to at least partially surround the patient insertion site including completely surrounding it. According to Fig. 1A, the top **56** substantially extends from the clasping means **51** to the compartment **70**. Optionally, the compartment **70** is in sealing contact with the seal of the patient contacting surface **41**. In one embodiment, the compartment **70** will include an effective amount of an antimicrobial (antiseptic) agent sufficient to minimize or eliminate infection at the insertion site. Suitable examples include certain alcohols and phenols known to have antiseptic properties (eg., isopropyl alcohol, phenol), iodine tinctures, chlorhexidine, povidone-iodine (Betadine®) and the like. Preferably, the agents are provided in the compartment 70 in a liquid, semi-liquid or cream format. Topical use antibiotics (eg., bacitracin zinc, polymysin B sulfate, and/or neomycin sulfate), preferably in cream form, can also be used alone or in combination with other antimicrobial agents.

In the embodiment shown in Figure 1A, the flexible base **20** is substantially planar. Preferably, the flexible base **20** is composed of a material (or composite) of sufficient rigidity so that self-folding is minimized or eliminated. To further minimize self-folding, the catheter clip **10** includes extensions **60**, **61**, and **62** of the solid portion **50** which are positioned to help keep the flexible base **20** substantially extended. Additional rigidity is provided by firming contact between the extensions **60**, **61** and **62** and the flexible base **20**.

Figure 1B shows the catheter clip **10** with a catheter **140** secured to the patient insertion site **90** which catheter **140** is composed of a catheter hard part **110** with connecting hubs **111**, **112**, lateral wings **100** for stabilizing the catheter and a catheter soft part **80** extending from the insertion site **90** toward and under the skin **120**. The catheter **140** is secured to the catheter clip **10** by actuation of clasping means **51** as previously described. The catheter clip **10** is secured to the patient through the adhesive of the patient contacting surface **41**.

Referring now to Figure 2, the catheter clip **10** is shown in cross-section with the catheter **140** secured to the patient insertion site **90.** Also shown is the top **56** of the solid portion **50** with a protrusion **130** that extends acutely from the axis **55** (see also Fig. 1A) and is in actuating contact with the clasping means **51**. The protrusion **130** is adapted to receive at least one digit from a user which digit engages protrusion **130** to flex the clasping means **51**, grasp the catheter **140** (preferably along the hard part **110**), and secure it to the catheter clip **10**.

According to one use of the catheter clip **10**, a caregiver inserts the soft part **80** of the catheter **140** under the skin **120** at the patient insertion site **90**. Subsequently, the caregiver uses a finger to remove cover **40** and the handle **30** in sealing contact with the adhesive along the patient contacting surface **41** of the flexible base **20**, thereby removing the seal and exposing the adhesive. The catheter clip **10** is then guided around the patient insertion site **90** to reversibly attach the invention to the skin **120** of the patient. Using the same or different finger, the caregiver engages the protrusion **130** to flex the clasping means **51** and grasp the hard part **110** of the catheter **140**, thereby releasably securing the catheter **140** to the catheter clip **10**. The procedure is readily reversible. In one approach, the patient contacting surface **41** is removed from the patient. The clasping means **51** is flexed by the user to allow removal of the catheter **140** from the invention.

### Manufacture

The invention can be readily made using one or a combination of approaches. In one method, the hard part of the catheter clip **10** is molded as a single-piece system with the clasping means **51** capable of convenient and repeated flexure. The hard part of the catheter clip **10** can be molded from a single polymer material in a one-shot injection molding operation. Many suitable materials could be used such as nearly any moldable plastic that is capable of being formed as provided herein and retaining its shape during use. More specific materials include, but are not limited to, many acrylic and polycarbonate materials, styrenes, and ABS. Other suitable materials include certain polyesters, nylons, and other polymer materials such as certain polyethylenes and polypropylenes. Certain memory plastics may also be used provided intended results are achieved. If two different materials are used, the device can be made by a two-shot process whereby both materials are injected at different gates into the mold cavity. Other processes are also feasible, such as insert molding. MRI compatible materials may be suitable for some invention applications.

Once the hard part of the catheter clip **10** is made, it can be joined using standard approaches to the soft part of the device which generally includes the flexible base **20**.

The flexible base **20** can be made using one or a combination of conventional approaches. In one method, the flexible base **20** comprises a relatively thin film, such as a thin urethane or silicone film, adhered to the handle **30** and cover **40**. The handle **30** and cover **40** can be made somewhat stiffer than the flexible base **20** facilitate handling. The handle **30** and the cover **40** may extend beyond an edge or edges of the thin film, so that the flexible base **20** can be handled relatively easily. In one embodiment, substantially all of the undersurface of the flexible base **20** (ie., the patient contacting surfacem41) is adhesively coated and contacted with a separate release seal which extends beyond the perimeter of the thin film and is in sealing contact with the handle **30** and cover **40**. Nearly any adhesive suitable for topical medical use can be applied to the thin film using standard methods. Examples include, but are not limited to, an acrylic adhesive containing an acrylic acid alkyl ester as a main component, a rubber adhesive containing a natural rubber and/or a synthetic rubber as a main component, as well as "pressure sensitive adhesives" (PSAs). See also U.S. Pat. Nos. 7,094,944; 6,936,661; and 6,805,961 and references disclosed therein for other suitable adhesives.

As will be apparent, the seal may protect the adhesive on at least part of the patient contacting surface 41 (e.g., less than about 50%, for instance, about 5%, 10%, 20% or about 30%), preferably substantially all of the surface (e.g., about 80%, 90%, 95% up to about 100%). The amount of adhesive on the patient contacting surface is not critical provided intended results are achieved.

### Additional Embodiments

As will be appreciated, other embodiments of the catheter clip **10** are within the scope of the invention. For instance, and referring now to Figure 1A, the clasping means **51** is shown as an integral part of the solid portion **50**. However in another embodiment, clasping means **51** may be joined to solid portion **50** through an elastic or semi-elastic polymer such as a memory plastic. Alternatively, the clasping means **51** can include or be joined to the solid portion **50** through a spring or related mechanism.

In another embodiment of the catheter clip **10**, the top **56** includes means (e.g., a spring or a rod) to position the clasping means **51** to a site desired by the user. It will be appreciated that in other embodiments, the top **56** and the clasping means **51** may be the same component.

In another embodiment, the compartment **70** can be quite small and be just sufficient to surround the insertion site of the catheter. In this embodiment, it will not be necessary to make the compartment **70** so that it is in sealing contact with other components of the device. Also in this embodiment, the use of an antimicrobial may not be necessary. Instead, it may be applied to the skin surface by the user.

Nearly any configuration of the protrusion **130** is acceptable provided intended results are achieved. For instance, and in one embodiment, the protrusion 130 will extend substantially perpendicular to the axis **55** of the open chamber 52.

In yet another embodiment of the catheter clip **10**, only one or two of the extensions **60**, **61**, and **62** will be used to provide rigidity to the flexible base **20**. In yet another embodiment, the flexible base **20** will be of sufficient rigidity so that none of extensions **60, 61** and **62** are needed.

### Equivalents

It will be appreciated that those skilled in the art, upon consideration of this disclosure, may make, modifications and improvements within the scope of the invention as defined in the claims. Moreover, those skilled in the art will recognize, or be able to ascertain using no more than routine experimentation, many equivalents of the specific embodiments of the invention described herein. Such equivalents are intended to be encompassed by the following claims.

## Claims

1. A device (10) for securing a catheter (140) to an insertion site (90) of a patient comprising:
(a) a flexible base (20) comprising a sealed patient contacting surface (41),
(b) a solid portion (50) joined to the flexible base (20) and comprising a clasping means (51) for securing the catheter (140); and
(c) a handle (30) adapted to remove a seal from the patient contacting surface (41), the handle (30) being joined to the seal, the seal protecting an adhesive on at least part of the surface (41) sufficient to attach the secured catheter (140) to the patient insertion site (90),
**characterized in that**
the solid portion (50), flexible base (20) and the handle (30) define an open chamber (52) comprising a distal end (53) for receiving the catheter (140) and a proximal end (54) for accepting the catheter (140) from the distal end (53) toward the patient insertion site (90);
the open chamber (52) comprises a top (56) defined by at least part of the solid portion (50), and a bottom (57) defined by the handle (30) and the flexible base (20), and
at least part of the top (56) comprises a protrusion (130) which is in actuating contact with the clasping means (51) such that actuation of the protrusion (130) is sufficient to flex the clasping means (51) so that it grasps and secures the catheter (140) to the device (10).

2. The device (10) of claim 1, wherein the distal end (53) of the open chamber (52) comprises a first centerpoint and the proximal end (54) comprises a second centerpoint, the open chamber (52) surrounding an axis (55) defined by the first and second centerpoints.

3. The device (10) of claim 1 or 2, wherein the flexible base (20) and optionally the solid portion (50) further define a compartment (70) at or near the patient insertion site (90), the compartment (70) being adapted to at least partially surround the site (90).

4. The device (10) of claim 3, wherein the top (56) extends from the clasping means (51) to the compartment (70).

5. The device (10) of claim 4, wherein the protrusion (130) extends acute to the axis of the open chamber (52).

6. The device (10) of claims 3to 5, wherein the compartment (70) is in sealing contact with the seal of the patient contacting surface (41).

7. The device (10) of claim 6, wherein the compartment (70) comprises a therapeutically effective amount of an antimicrobial agent to minimize infection at the insertion site (90).

8. The device (10) of claims 1 to 7, wherein the flexible base (20) is planar and of sufficient rigidity to minimize self-folding.

9. The device (10) of claim 8, wherein the rigidity is provided by firming contact with one or more extensions (60, 61, 62) of the solid portion (50).

10. A unitary package comprising the device (10) of claims 1 to 9, wherein the device (10) is sterile and comprises a packing material resistant to penetration by microorganisms.

## Patentansprüche

1. Vorrichtung (10) zum Sichern eines Katheters (140) an einer Einbringstelle (90) eines Patienten, mit:
(a) einer flexiblen Basis (20) mit einer versiegelten Patientenkontaktfläche (41),
(b) einem soliden Abschnitt (50), der mit der flexiblen Basis (20) verbunden ist und eine Klammereinrichtung (51) zum Sichern des Katheters (140) aufweist; und
(c) einer Ergreifeinrichtung (30), die angepasst ist, um eine Versiegelung von der Patientenkontaktfläche (41) zu entfernen, wobei die Ergreifeinrichtung (30) mit der Versiegelung verbunden ist, und wobei die Versiegelung einen für die Befestigung des gesicherten Katheters (140) an der Patienteneinbringstelle (90) ausreichenden Klebstoff an wenigstens einem Teil der Oberfläche (41) schützt,
**gekennzeichnet dadurch, dass**
der solide Abschnitt (50), die flexible Basis (20) und die Ergreifeinrichtung (30) eine offene Kammer (52) festlegen, die ein distales Ende (53) zum Empfangen des Katheters (140) und ein proximales Ende (54) zum Annehmen des Katheters (140) von dem distalen Ende (53) aus in Richtung hin zu der Patienteneinbringstelle (90) aufweist;
die offene Kammer (52) eine durch wenigstens einen Teil des soliden Abschnitts (50) festgelegte Decke (56) und einen durch die Ergreifeinrichtung (30) und die flexible Basis (20) festgelegten Boden (57) aufweist, und
wenigstens ein Teil der Decke (56) einen Vorsprung (130) aufweist, der sich in Betätigungskontakt mit der Klammereinrichtung (51) befindet, so dass eine Betätigung des Vorsprungs (130) ausreichend ist, um die Klammereinrichtung (51) so zu biegen, dass diese den Katheter (140) greift und an der Vorrichtung (10) sichert.

2. Vorrichtung (10) nach Anspruch 1, wobei das distale Ende (53) der offenen Kammer (52) einen ersten Mittelpunkt aufweist und das proximale Ende (54) einen zweiten Mittelpunkt aufweist, wobei die offene Kammer (52) eine Achse (55) umgibt, die durch den ersten und den zweiten Mittelpunkt festgelegt ist.

3. Vorrichtung (10) nach Anspruch 1 oder 2, wobei die flexible Basis (20) und optional der solide Abschnitt (50) ferner einer Raum (70) an oder nahe der Patienteneinbringstelle (90) festlegen, wobei der Raum (70) angepasst ist, um wenigstens teilweise die Stelle (90) zu umgeben.

4. Vorrichtung (10) nach Anspruch 3, wobei sich die Decke (56) von der Klammereinrichtung (51) zu dem Raum (70) erstreckt.

5. Vorrichtung (10) nach Anspruch 4, wobei sich der Vorsprung (130) im spitzen Winkel zu der Achse der offenen Kammer (52) erstreckt.

6. Vorrichtung (10) nach einem der Ansprüche 3 bis 5, wobei der Raum (70) in dichtendem Kontakt mit der Versiegelung der Patientenkontaktfläche (41) steht.

7. Vorrichtung (10) nach Anspruch 6, wobei der Raum (70) eine therapeutisch wirksame Menge an antimikrobiellem Mittel zur Infektionsminimierung an der Einbringstelle (90) aufweist.

8. Vorrichtung (10) nach einem der Ansprüche 1 bis 7, wobei die flexible Basis (20) eben ist und ausreichend Steifigkeit aufweist, um ein Selbstfalten zu minimieren.

9. Vorrichtung (10) nach Anspruch 8, wobei die Steifigkeit durch festigenden Kontakt mit einer oder mehreren Fortsätzen (60, 61, 62) des soliden Abschnitts (50) gegeben ist.

10. Einheitspackung mit der Vorrichtung (10) nach einem der Ansprüche 1 bis 9, wobei die Vorrichtung (10) steril ist und ein Verpackungsmaterial aufweist, das resistent gegenüber dem Eindringen durch Mikroorganismen ist.

## Revendications

1. Dispositif (10) pour fixer un cathéter (140) à un site d'insertion (90) d'un patient, comprenant :
(a) une base flexible (20) comprenant une surface scellée (41) d'entrée en contact avec le patient,
(b) une partie solide (50) jointe à la base flexible (20) et comprenant un moyen d'accrochage (51) pour fixer le cathéter (140) ; et
(c) une poignée (30) adaptée pour éliminer un scellement de la surface (41) d'entrée en contact avec le patient, la poignée (30) étant jointe au scellement, le scellement protégeant un adhésif sur au moins une partie de la surface (41) suffisante pour attacher le cathéter fixé (140) au site d'insertion (90) du patient,
**caractérisé en ce que**
la partie solide (50), la base flexible (20) et la poignée (30) définissent une chambre ouverte (52) comprenant une extrémité distale (53) pour recevoir le cathéter (140) et une extrémité proximale (54) pour accepter le cathéter (140) depuis l'extrémité distale (53) vers le site d'insertion (90) du patient ;
la chambre ouverte (52) comprend un haut (56) défini par au moins une partie de la partie solide (50), et un bas (57) défini par la poignée (30) et la base flexible (20), et
au moins une partie du haut (56) comprend une protubérance (130) qui est en contact d'actionnement avec le moyen d'accrochage (51) de sorte que l'actionnement de la protubérance (130) soit suffisant pour faire fléchir le moyen d'accrochage (51) pour qu'il saisisse et fixe le cathéter (140) au dispositif (10).

2. Dispositif (10) selon la revendication 1, dans lequel l'extrémité distale (53) de la chambre ouverte (52) comprend un premier point central et l'extrémité proximale (54) comprend un second point central, la chambre ouverte (52) entourant un axe (55) défini par les premier et second points centraux.

3. Dispositif (10) selon la revendication 1 ou 2, dans lequel la base flexible (20) et facultativement la partie solide (50) définissent en outre un compartiment (70) au, ou près du, site d'insertion (90) du patient, le compartiment (70) étant adapté pour entourer au moins partiellement le site (90).

4. Dispositif (10) selon la revendication 3, dans lequel le haut (56) s'étend du moyen d'accrochage (51) au compartiment (70).

5. Dispositif (10) selon la revendication 4, dans lequel la protubérance (130) s'étend selon un angle aigu par rapport à l'axe de la chambre ouverte (52).

6. Dispositif (10) selon les revendications 3 à 5, dans lequel le compartiment (70) est en contact de scellage avec le scellement de la surface (41) d'entrée en contact avec le patient.

7. Dispositif (10) selon la revendication 6, dans lequel le compartiment (70) comprend une quantité thérapeutiquement efficace d'un agent antimicrobien pour minimiser l'infection au site d'insertion (90).

8. Dispositif (10) selon les revendications 1 à 7, dans lequel la base flexible (20) est plane et de rigidité suffisante pour minimiser l'auto-repliement.

9. Dispositif (10) selon la revendication 8, dans lequel la rigidité est fournie par contact raffermissant avec un ou plusieurs prolongements (60, 61, 62) de la partie solide (50).

10. Conditionnement unitaire comprenant le dispositif (10) selon les revendications 1 à 9, dans lequel le dispositif (10) est stérile et comprend un matériau de conditionnement résistant à la pénétration par des microorganismes.
